(19)

Europäisches
Patentamt

European
Patent Office

Office européen
des brevets

(11)  EP 4 368 941 A1

(12)  EUROPEAN PATENT APPLICATION

(43) Date of publication:
**15.05.2024  Bulletin 2024/20**

(21) Application number: **22207284.5**

(22) Date of filing: **14.11.2022**

(51) International Patent Classification (IPC):
**G01B 7/16** (2006.01)    **A61B 5/11** (2006.01)
**A61B 5/00** (2006.01)    **D03D 1/00** (2006.01)
**G01L 1/14** (2006.01)    **G01L 1/22** (2006.01)
**G01L 9/12** (2006.01)    **G01P 15/125** (2006.01)
**G01R 15/18** (2006.01)

(52) Cooperative Patent Classification (CPC):
**G01B 7/18; A61B 5/1116; A61B 5/112;**
**A61B 5/1126; A61B 5/6804; G01B 7/22;**
**G01L 1/142; G01R 15/181;** A61B 5/27;
A61B 2562/0261

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **ETH Zurich**
**8092 Zurich (CH)**

(72) Inventors:
• **MENON, Carlo**
 **Zürich 8001 (CH)**
• **CUTHBERT, Tyler**
 **8008 Zürich (CH)**
• **GALLI, Valeria**
 **8005 Zürich (CH)**
• **AHMADIZADEH, Chakaveh**
 **8008 Zürich (CH)**
• **ROBERJOT, Pierre**
 **69420 Tupin et Semons (FR)**

(54)  **NOVEL SENSORS SUITABLE FOR MONITORING MOVEMENT AND OTHER BIOPHYSICAL PARAMETERS**

(57)  The invention relates to the field of mobile health technology and activity monitoring and refers in particular to sensors suitable to be used for wearable electronic devices. The first aspect of the invention refers to flexible or stretchable sensors that can be seamlessly incorporated into textiles, clothing, and wearable devices. A second aspect of the invention refers to a sensor using helical-auxetic yarns for construction of a capacitor. The sensors can also be stretchable and are suitable for use in textiles and for sensing body motion (strain).

Fig. 1

EP 4 368 941 A1

**Description**

FIELD OF THE INVENTION

The invention relates to the field of mobile health technology and activity monitoring and refers in particular to sensors suitable to be used for wearable electronic devices. The first aspect of the invention refers to flexible sensors that can be seamlessly incorporated into textiles, clothing, and wearable devices. A second aspect of the invention refers to a sensor using helical-auxetic yarns for construction of a capacitor. The sensors are also suitable for use in textiles and for sensing body motion (strain).

BACKGROUND OF THE INVENTION

Mobile health monitoring represents a breakthrough in the healthcare paradigm, as it opens the possibility for personalized and remote healthcare monitoring and risk assessment. The key value of mobile health technologies is the acquisition of relevant physiological data in a naturalistic, unconstrained, and ideally unperceived manner. Solutions should acquire and transmit data without restricting motion and should be comfortable to use; textile/clothing-based solutions present an opportunity to fulfil these criteria and advance how and where we obtain important bio-signal data. Wearables can be broadly subdivided into three categories: accessories like smartwatches, wristbands, earphones, and glasses; devices in direct contact with the body like epidermal devices (skin patches), contact lenses, tooth patches; and devices integrated into garments. Recent efforts in the third category have focused on textile-based wearables that incorporate functionalized textile elements into the garment with the goal of turning clothing into smart devices. The advantages of textile-based wearables are their flexibility and ability to form to the body, their lightweight and comfort, and their customizability. Eliminating the rigid power source, rigid electronics, and wired connection from textile-based wearables enables more seamless integration and greater capabilities for tracking motion and bio-signals, which in turn can potentially enhance user adaptation for daily use. Functionalized garments have served to interconnect multiple sensor tags in different locations on a garment. So far, most solutions use silicon chips on the skin as the sensors. Recent attempts to use textile near-field communication (NFC) radio-frequency identification (RFID) antennas move in the direction of all-textile sensor tags, but a silicon chip is still required at this time for communication. One goal of the present invention is therefore the development of all-textile sensors, electrical components/connections, and communication as part of the garment itself.

There were efforts integrating wireless sensing platforms in textiles include custom made sensing platforms with inductance-capacitance (LC) circuitry that rely on resonance frequency shifts for signal detection. Materials used to provide common fabrics with electrical conductivity are known, using different techniques to confer conductivity to standard fabrics at the level of single fiber, yarn (filament), or fabric. Inductive coupling has been used as a simple short-range wireless readout. In these studies, the change in capacitance resulted in a shift in the resonance frequency of the LC circuit; sensors employed have included capacitors measuring humidity and pressure, although an additional amplification and signal transmission circuit was required. Expansion of this passive wireless sensing technology to include fully textile sensing modalities is required to realize bio-sensing and movement tracking such as accurate motion tracking (i.e. joint angles), respiration rate, or heart rate. In addition, advancing alternative sensing modalities that are inherently textile would expand the possible tools for applied researchers looking to employ wearable devices in rehabilitation, performance improvement, injury prevention, and well-being.

One solution can be flexible and stretchable soft sensors in fiber forms. These are attractive for wearable device applications since they can be integrated directly as part of the textiles that make up our clothing and turn it into multi-functional devices. A key drawback of integration of stretchable sensors is not a trivial pursuit because the mismatch of mechanical properties between sensors and textiles. Large differences in mechanical properties can cause issues with weaving or knitting and may cause irreversible damage to the sensors during fabrication. Flexible fiber sensors typically come in three kinds-piezoresistive (the most prominent modality), inductive, and capacitive. The capacitive sensing mechanism has a distinct advantage that it relies only on geometry; this feature eliminates any issues (signal inconsistency, drift, hysteresis, etc) relating to the percolating conductive network or degradation of the rigid electrode on a flexible/stretchable substrate often employed in piezoresistive sensors.

It is therefore another goal of the present invention to develop a flexible fiber sensor having a capacitive sensing mechanism with a sensitivity within the working strain range for textiles and wearable devices. The inventors were able to achieve a gauge factor above the theoretical gauge factor limit of 1 for capacitive sensors that employ classical materials restricted by Poisson's ratio. They could show that specific auxetic structures have the potential to increase the sensitivity of capacitive sensors, especially as sensors are downsized and take on structures/morphologies that are more amenable to integration into wearable devices such as textiles.

SUMMARY OF THE INVENTION

It is an object of the invention to provide a sensor for detection of strain comprising a LC circuit and being made using exclusively textile components. Thereby a LC circuit, also called a resonant circuit, tank circuit, or tuned circuit, is an electric circuit comprising an inductor, represented by the letter L, and a capacitor, represented by the letter C, connected together. The circuit can act as an electrical resonator, an electrical analogue of a tuning fork, storing energy oscillating at the circuit's resonant frequency.

It is an object of the invention to provide a sensor for detection of strain using exclusively textile components. This means the invention refers to a sensor for detection of strain comprising a LC circuit which consists only of textile components. Monitoring strain is suitable to track body motion as it conveniently aligns with natural deformation of tight-fitting clothes during movement, and it can be easily adapted to monitor motion of different parts of the body. The results of the present invention indicate that textile-based sensors have the capabilities to combine the advantageous effects of capacitive sensors with greater sensitivities required to increase the accuracy of wearable devices for broader implementation, use, and acceptance.

Textile as used herein refers to various fiber-based materials such as fibers, yarns, filaments, threads, and fabrics. A fabric is defined as any thin, flexible material made from yarn, directly from fibers, polymeric film, foam, or any combination of these techniques. A fiber is the smallest component of a fabric; fibers are typically spun into yarn, and yarns are used to manufacture fabrics by weaving, knitting, crocheting, knotting, tatting, or braiding. The fiber has a hair-like appearance and a higher length-to-width ratio. The sources of fibers may be natural, synthetic, or both. After manufacturing, textile materials may be processed and finished to add value, such as aesthetics, physical characteristics, and increased usefulness.

One embodiment of the invention refers to a sensor for detection of strain consisting of a passive LC resonator. The sensor according to the invention may be composed of a textile capacitor sensitive to strain, an inductor made of a textile (in particular a fixed value inductor), and an electrical connection between these two, forming a resonant circuit. It is preferred that the sensor according to the invention comprises no batteries, silicon or elements made of polychlorinated biphenyl. Therefore, one embodiment refers to a sensor or sensor system consisting of flexible components being made without rigid or silicon-based parts. This allows to produce an unobtrusive and comfortable garment, such as a pair of tights/pants, that wirelessly track body movement.

It is further preferred that the sensor according to the invention is flexible. This means the sensor is capable to bend or to be bent easily without breaking. In other words, it is pliable. It is further preferred that the sensor is elastic. This means the sensor or respectively the material the sensor is made from is able to deform elastically and return to its original shape when the applied stress is removed. Thereby, elasticity is the ability of a body to resist a distorting influence and to return to its original size and shape when that influence or force is removed. Thus, the sensors may be easily resuming its original size or shape after being stretched or otherwise deformed. It is preferred that the material, the sensor or parts therefrom are made of have a Young's modulus between 1 and 10 kPa and preferably about 3 kPa.

In another embodiment the sensor according to the invention is made of a textile, in particular a fabric, that stretches (stretch fabrics, in particular synthetic stretch fabrics). Stretch fabrics can either be 2-way stretch or 4-way stretch. 2-way stretch fabrics stretch in one direction, usually from selvedge to selvedge (but can be in other directions depending on the knit). 4-way stretch fabrics stretches in both directions, crosswise and lengthwise. Preferred stretch fabrics suitable as material for the sensors according to the present invention are fabrics consisting of or containing spandex or elastane (widely branded as "Lycra"). It is a polyether-polyurea copolymer. To produce a textile suitable for the present sensor, the elastane fibers can be mixed with cotton or polyester, and may account for a minor percentage of the final fabric.

In another embodiment of the invention the inductor of the LC circuit comprises a sewn pattern of a conductive thread or is designed as a sewn pattern of a conductive thread. A conductive textile is a thread which can conduct electricity. Conductive fiber or a conductive thread may consist of a non-conductive or less conductive substrate, which is then either coated or embedded with electrically conductive elements, often carbon, nickel, copper, gold, silver, titanium or poly-3,4-ethylendioxythiophen (PEDOT). Preferred are threads made of a polymer inner layer surrounded by a metal outer layer. The conductive yarns, threads, or fibers can be used as monofilaments, multifilaments, and as twisted yarns. Low resistance threads are particularly suitable. Suitable are for example silver-plated polyamide yarns, a silver-coated vectran thread or high conductivity stainless steel threads ("Bekinox" technology from Bekaert).

Conductive textiles or fabrics are textiles or respectively fabrics which can conduct electricity. They can be made with metal strands woven into the construction of the textile or by conductive yarns which are conductive thanks to a metal-coating. Also suitable are textiles, made by impregnating normal textiles with carbon- or metal-based powders.

The invention concerns further a sensor, wherein the capacitor of the LC circuit has a double plate capacitor configuration. In particular, the capacitor consists of two conductive, flexible sheets of a textile material separated by at least one layer of a non-conductive flexible textile material. Thereby a sheet of textile material refers to a thin layer of a textile. The sheet of a textile may be a thin, flat, and rectangular piece of elastic fabric, more preferred a stretchable fabric. In particular, the sheet can be deformed in directions parallel to the surface of the sheet and return to their original shape and size after the forces deforming it have been removed. The deformation results in an extension of the sheet in at least one direction. The deformation/extension is thereby directly proportional to the force working. The invention concerns further a sensor, wherein the capacitor of the LC circuit is made comprising two sheets of conductive elastic or stretchable fabric, e.g. spandex containing fabric, separated by two layers of non-conductive elastic or stretchable fabric e.g. spandex containing fabric. It is advantageous if the conductive and the non-conductive (isolating) fabric used for the capacitor have similar mechanical properties in particular have a similar stretchability.

The textile inductor comprises or consists of a sewn pattern of a conductive thread, such as a rectangular or oval loop. The loop may contain between 3 and 10 turns, preferably between 4 and 7 turns and most preferred it is made with 5 turns. It is preferred that the gap between the turns is consistent. It may be a 0.5 to 3 and preferably a 2 mm gap, wherein the gap refers to the distance between each loop of the textile inductor. A consistent gap between the loops can be ensured during fabrication. It is advantageously, that the inductor is flat (without wrinkles). It is therefore possible to use wash-away sewing stabilizer to avoid wrinkles.

In one embodiment of the invention the capacitor and the inductor of the sensor are connected using a conductive thread. This thread may be sewn on a textile. It may be the same textile used as isolating layer between the "plates" or conducting sheets of the capacitor. Also, the pattern of the inductor may be sewn on the same textile. The conductive thread serves as connection between the capacitor and the inductor to close the LC circuit. The conductive thread may build a transmission line running vertically between both elements. In particular it can be sewn forming a zigzag to allow free movement of the body and be able to stretch if required. The transmission line length may be between 15 and 300 mm.

In one embodiment of the invention the inductor consists of a sewn pattern of a conductive thread, whereas the capacitor is composed with a stretchable conductive textile; and both are connected through a conductive fiber. The sensor is capable of tracking strain through a resonance frequency modulation resulting from capacitance changes in response to strain/movement.

One embodiment of the present invention refers to a sensor being arranged to enable wireless readout. The present application refers therefore further to a system comprising a sensor as described herein and a transponder unit configured to measure a frequency response of the sensor. One embodiment of the present invention refers to a system, wherein the readout is performed via inductive coupling of an external inductive coil. This allows to read the data wirelessly through inductive coupling of the transponder unit to the inductor of the sensor. Therefore, a transponder unit configured to measure a frequency response of the sensor can be used. It is preferred that the system allows to place the transponder unit on top of the LC circuit and in particular on the inductor. This means the system is arranged so that the transponder unit and the inductor are located parallel or respectively adjacent. It is preferred that the axial distance between the two inductors, is less than 10 mm, 25 mm or 50 mm. In case the inductor has a rectangular shape, a radial distance (displacement in the-plane direction) should be less than half of the inductor size.

Such systems can employ inductive coupling to read wirelessly and passively (i.e. no battery power) with LC resonators as sensors, thus avoiding the use of all rigid silicon-based components through the use of textile-based electronics. One possible transponder unit for wireless readout may be a portable vector network analyzer that detected the resonance frequency of the LC resonator in real time. The transponder unit can communicate with a mobile phone as external communication device.

Because the sensors of the invention are full-textile sensors able to be used together with wireless communication they can be integrated into essentially any piece of clothing and in particular into a motion tracking garment.

Therefore, the present invention refers further to a garment including a sensor as described herein. Such a garment may further comprise a transponder unit configured to measure a frequency response of the sensor. The transponder unit may be located in a convenient pocket of the garment.

A garment comprising a sensor of the invention is useful for accurately tracking body movement. Thus, it is suitable for health monitoring. One embodiment of the present invention refers to the use of a sensor as described herein for tracking human motion.

The conductive sheets forming the plates of the capacitor can take any shape and be any size. However, it is preferred that it has a geometry or dimension that allows the sensor to be included in a garment. The plate capacitor may have, for instance, the shape of a complete pant leg. In addition, the sheets shape should be overlapping. It is advantageously, that the distance between the conductive sheets is the lowest possible.

The sheets forming the plates of the capacitor (made of conductive textile) can have a size between 750 and 3000 mm², in particular between 1000 and 2000 mm². The sheet can have a length and with between 15 and 200 mm, preferably between 25 and 100 mm and in particular between 30 and 75 mm. They may be approximately 50 mm x 35 mm in size. The sheets forming the plates of the capacitor can have a thickness between 0.005 and 5 mm, in particular between 0.2 and 0.5 mm.

In another embodiment of the invention the inductor of the LC circuit comprises a sewn pattern of a conductive thread. The thread can be sewn in a rectangular loop or an oval loop. The length and width of the loop can be between 40 and 150 mm. In principle the inductor can have any size which fits on the garment. However, within a system, wherein the readout is performed via inductive coupling of an external inductive coil, ideal coupling occurs when the inductor of the LC circuit and the external inductor have the same size. Preferred dimensions are therefore 80 mm x 60 mm.

The sensors as described herein are ablet to track human motion as a resonance frequency modulation resulting from capacitance changes in response to strain/movement. The resonance frequency of the circuit can be tracked as the capacitance changed during physical movement. The modulation of the resonance frequency occurs in response to a change in geometry of the capacitance. The change in geometry of the capacitance may be caused by stretch or bending of the conducting sheets the capacitor can be made of.

The inventors could further develop a capacitive strain sensor that was capable of accessing greater sensitivity and is formed as a fiber for direct integration into textiles. These sensors comprise a helical auxetic yarn working as a capacitor e.g. within an LC circuit. This, the present invention refers to the use of a helical auxetic yarn for a capacitive sensor (auxetic capacitive sensor). Therefore, a second aspect of the invention refers to a sensor comprising a capacitor formed by a helical auxetic yarn. This auxetic capacitive sensors is suitable for use in textiles and/or for sensing body motion (strain). Therefore, one embodiment of the present invention relates to a sensor for detection of strain comprising an auxetic capacitive sensor being made using exclusively textile components.

The term "helical auxetic yarn" refers to a non-extending or not stretchable fiber helically wrapped around an elastic fiber. In addition to this, an insulating material would be required between both fibers. The elastic fiber is capable of being easily stretched or expanded and resuming former shape. It can in particular be extended in length and is capable of returning to its original length, when the pulling force ends. The fiber being non-extending or not stretchable cannot be extended in length, at least not without destruction or irreversible deformation. The insulating material can be any insulating material which does not interfere with the auxetic characteristic of the fiber. One possibility is an enamel coating of the non-extending fiber but also a polymer coating of the elastic fiber may be suitable as long as elasticity is not impaired.

It is preferred that the non-extending fiber as well as the elastic fiber are conductive. One embodiment of the present invention refers to the auxetic capacitive sensor, wherein the helical auxetic yarn is made from at least two conductive fibers twisted around one another and insulated from one another and wherein the first fiber of the at least two conductive fibers is non-extending and the second fiber of the at least two conductive fibers is elastic. The non-extending fiber may be malleable but non-extending.

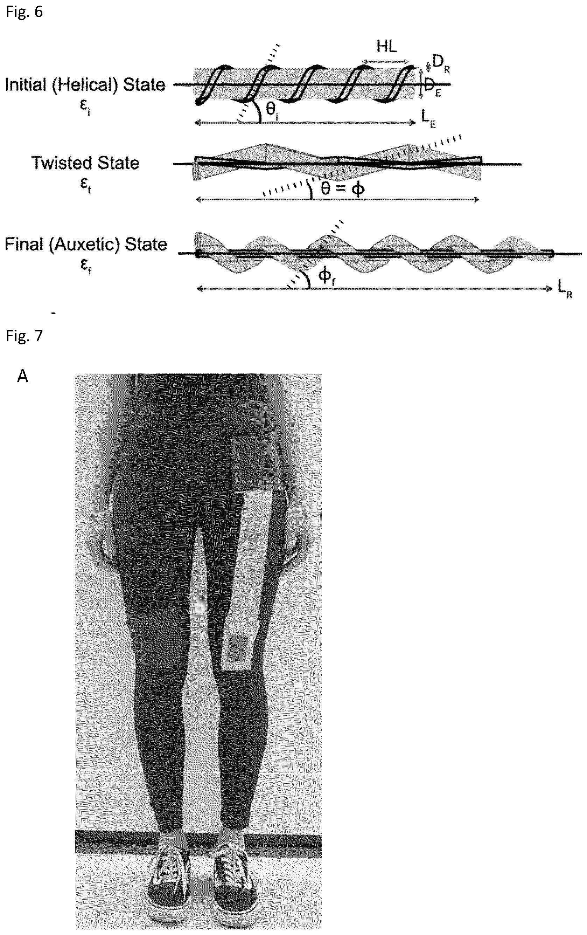

Therefore, one embodiment of the invention refers to a sensor comprising a capacitor formed by a helical auxetic yarn which is composed of a conductive non-extending fiber of a defined length (LR), a defined diameter (DR), wrapped around a conductive elastic fiber of defined length (LE), defined diameter (DE) with a specific pitch (either as an angle, $\theta$, or simply a length associated with a complete helix, PR). Without a force acting, the non-extending fiber and the longitudinal axis of the helix forms an angle theta which decreases as strain is applied. The angle between the elastic fiber and the longitudinal axis is zero to begin and increases as strain is applied (see Figure 6).

The auxetic effect of the sensor induces a geometrical change in the |cross-sectional area of the sensor, which in turn increases capacitance at a greater rate than the change in length (strain) leading to a gauge factor greater than the theoretical limit of 1. Therefore, one embodiment of the sensor according to the second aspect refers to an auxetic capacitive sensor having a gauge factor of > 1. These sensors are particularly suitable for use in textiles and/or for sensing body motion (strain). Another embodiment of the invention refers to a sensor for detection of strain comprising a LC circuit and being made using exclusively textile components, wherein the capacitor of the LC circuit comprises a helical auxetic yarn. One embodiment refers to said sensor, wherein the helical auxetic yarn is made from at least two conductive fibers twisted around one another and insulated from one another and wherein the first fiber of the at least two conductive fibers is non-extending and the second fiber of the at least two conductive fibers is elastic. It is preferred that the ratio of diameter between the first and the second fiber or respectively the non-extending and the elastic fiber is between 1:5 and 1:50. It is even more preferred that said ratio is between 1:8 and 1:30 or 1:10 and 1:20. Thereby the fibers have a diameter between 50 and 1000 $\mu$m.

One embodiment of the auxetic capacitive sensor of the invention has a wrapping angle < 40°. This angle is measured in an initial phase which means that now force is acting on the auxetic helical yarn of the sensor. By changing the ratio of the fiber diameter and the angle the second fiber is wrapped around the first fiber, the gauge factor of the auxetic capacitive sensor of the invention is tunable. All these statements about the dimension of the helical auxetic yarn refer to the situation where the elastic fiber of the yarn is in the relaxed state. In other words, no force is acting on the helical auxetic yarn or components thereof which lengthen or stretch the yarn.

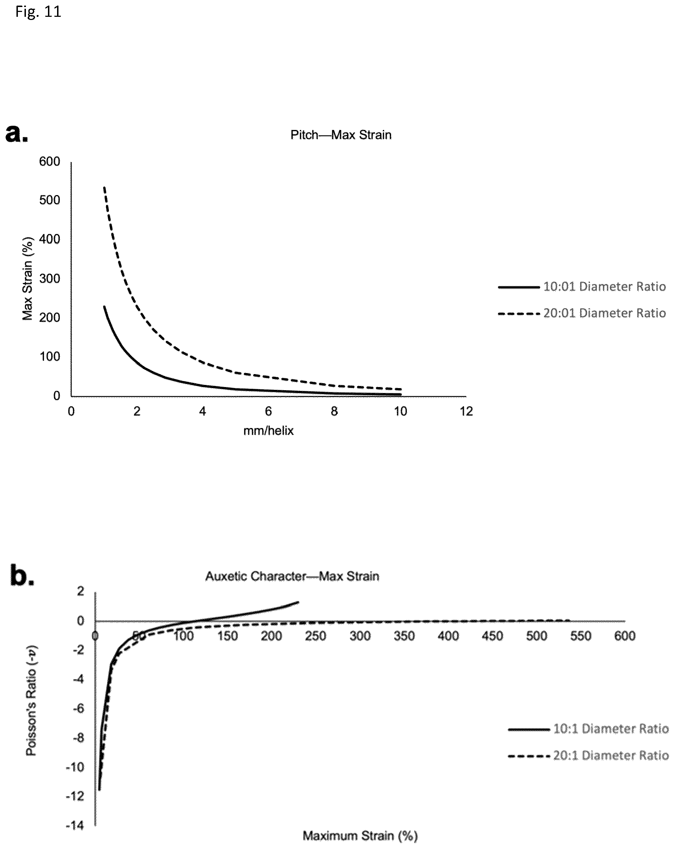

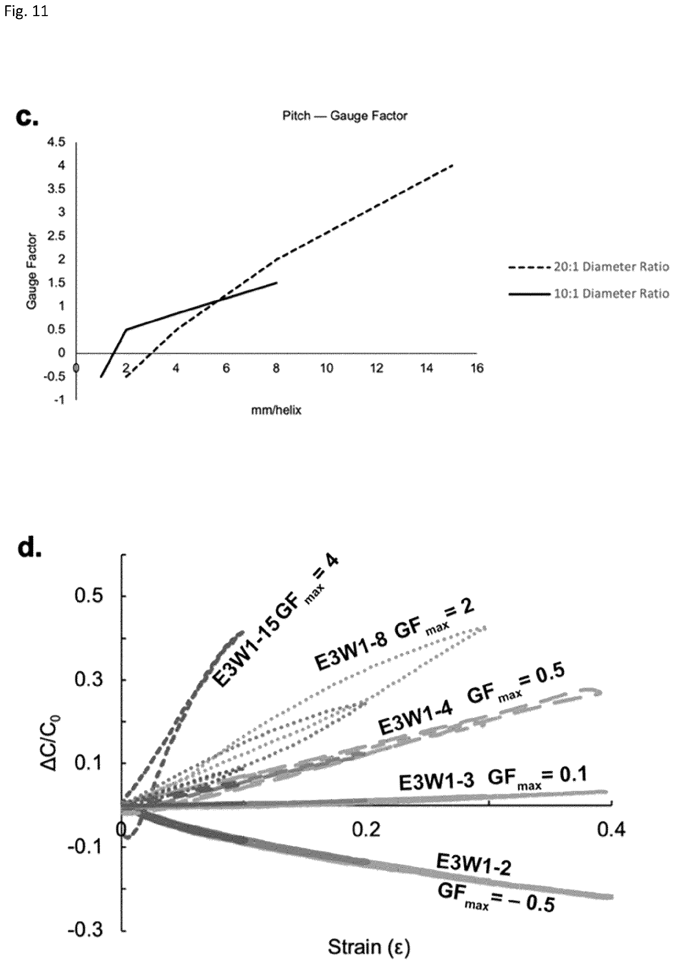

The auxetic capacitive sensor of the invention may be variable in the diameters of the wire used and the twisting angle of the non-extending wire around the inner elastic wire. The diameter of the non-extending wire can be as small as possible that allows the mechanical deformation of the elastic wire. It can be between 10 $\mu$m and 5 mm in diameter. Its size can be adapted to the fabric, into which the sensor is integrated. More important is the ratio of the diameters between the fibers. The larger the ratio, the easier it is to access auxetic character. Preferred ratios which have been found to be suitable are between 10:1 and 20:1 (Fig. 11). Hence the non-extending wire may have a diameter within the range of 50 to 200 $\mu$m and the elastic wire may have a diameter between 1 to 3 mm.

The wires can be wound as tightly as possible (i.e. have a high pitch angle, or a short helical length) to as straight as possible (i.e. the lowest pitch angle, or the longest helical length) depending on the combination of the wire's diameters. The twisting angle which can also be termed as the helical length or screw pitch (which would be described in mm / helix) can be greater than 0° and less than 90°. A larger pitch angle (shorter helical length) would result in a greater maximum strain and a larger negative gauge factor (Fig. 11). A smaller pitch angle would result in a lower maximum strain and a larger positive gauge factor (above the theoretical limit, Fig. 11).

BRIEF DESCRIPTION OF THE DRAWINGS

Exemplarily embodiments of the invention are shown in the following figures. In the figures, the same reference symbol is used for identical or comparable elements. It shows:

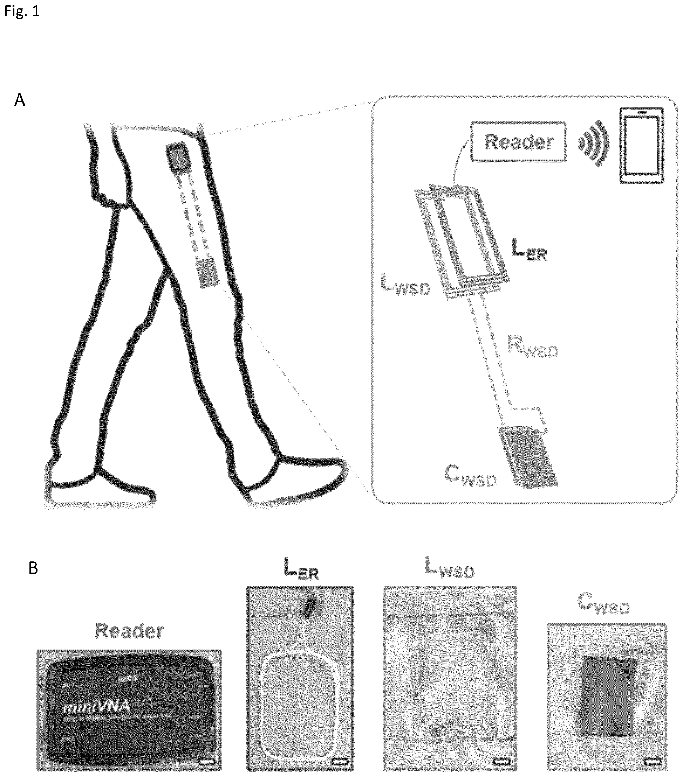

Figure 1: shows in part A a schematical drawing of one system according to the invention comprising an LC circuit, a transponder unit and a reader used to transfer the signals to a portable device. The LC circuit is composed of a double plate textile capacitive sensor (CWSD, in blue) was mounted above the kneecap and was connected to a textile inductor (LWSD, in green) via a transmission line (grey dashed lines). The transmission line together with the ESR of the textile inductor make up the overall resistance of the circuit (RWSD, in grey). LWSD inductively couples with a fixed inductor LER (in red) that was connected to a commercial portable reader (in light green). The transponder unit (e.g. VNA reader) can communicate wirelessly with a smartphone. All scalebars in the pictures are 1 cm. B shows photographs of the different parts of an exemplarily tested system according to the invention. $L_{ER}$ refers to an inductor being part of the transponder unit.

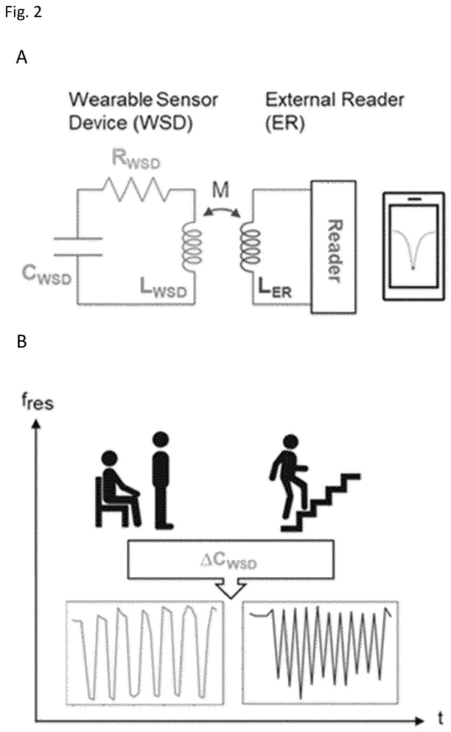

Figure 2 shows in part A an electrical circuit equivalent to the system shown in Figure 1 and schematic representation of the signal read. Overview of the system. a, Schematic illustration of the system. B shows a representative graph for activity recognition in two different scenarios (sit-stand-sit and stair climbing).

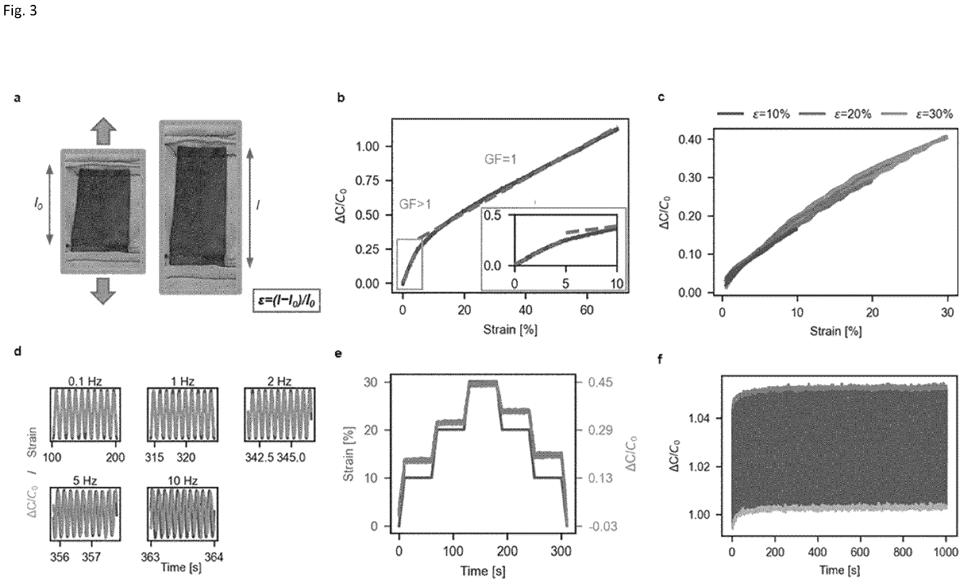

Figure 3 shows the mechanical-electrical characterization of a textile capacitive sensor in response to strain. a, Image of CWSD, example representation of a uniaxial tensile test, and the expression for the calculation of strain. b, Capacitance change during strain to 30% and the accompanying gauge factor. c, Hysteresis test with strain from 0 to 3%, 6% and 9% strain. d, Sensor bandwidth tests with increasing strain frequency. e, Drift of the capacitance when held at specific strain values. f, Drift of peaks and valleys during long term stability test of 1000 cycles.

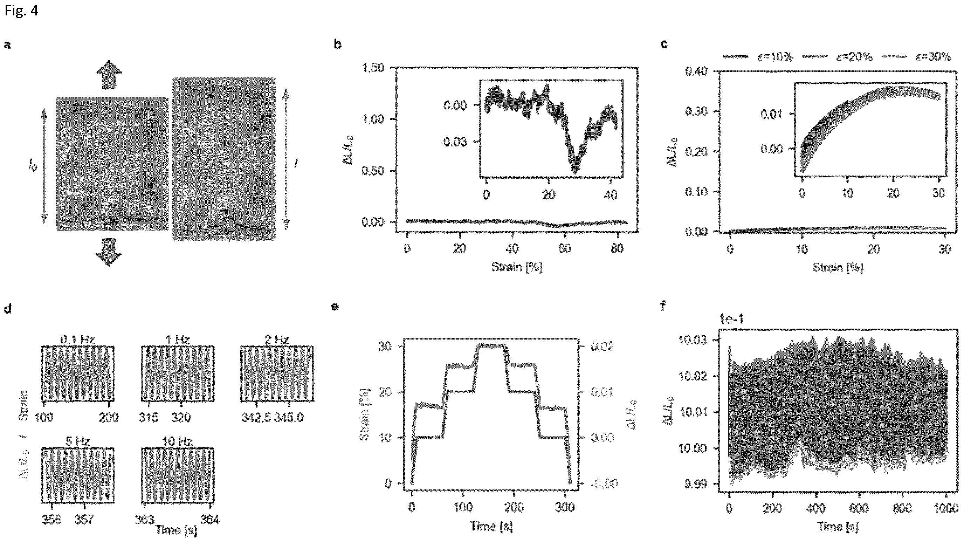

Figure 4 shows a mechanical-electrical characterization of LWSD. a, Image of the LWSD, example representation of a uniaxial tensile test, and expression for the calculation of strain. b, Inductance during strain to 75%. c, Hysteresis test with strain from 0 to 10%, 20% and 30% strain. d, Sensor bandwidth from tests at increasing strain rates. e, Drift of the capacitance when held at specific strain values. f, Drift of peaks and valleys during long term stability test of 1000 cycles.

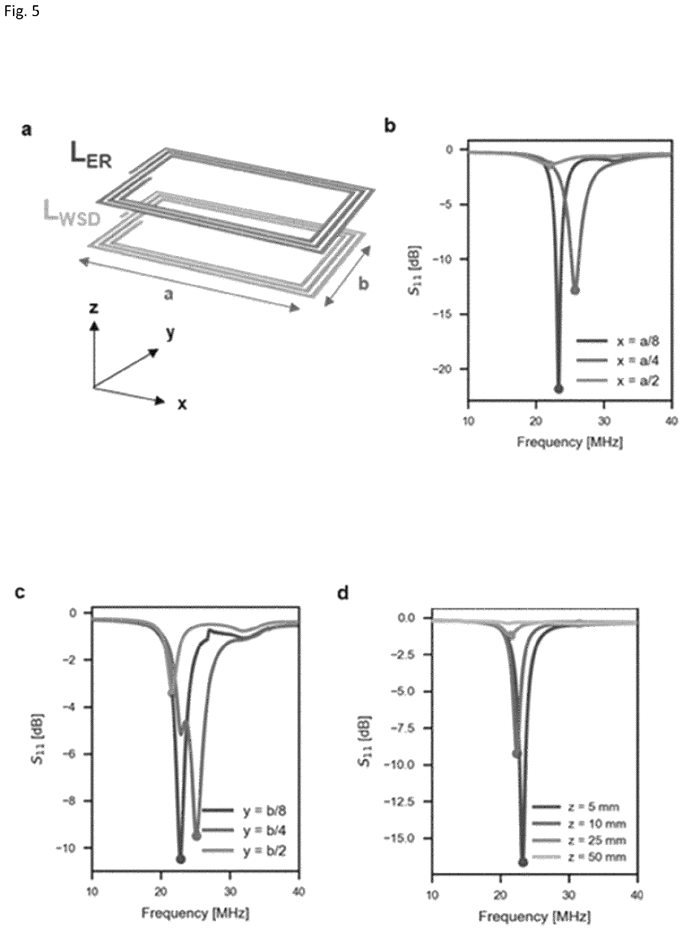

Figure 5 shows Influence of the reciprocal position of L_S and L_R on wireless reading. a, Schematic of the tests. b, c, Influence of horizontal displacement. d, Influence of vertical displacement.

Figure 6 shows a) a geometrical representation of a helical auxetic yarn in three different states of the sensor, the initial state, the twisted state, where the two fibers have the same length and the final state where the auxetic behavior happens.

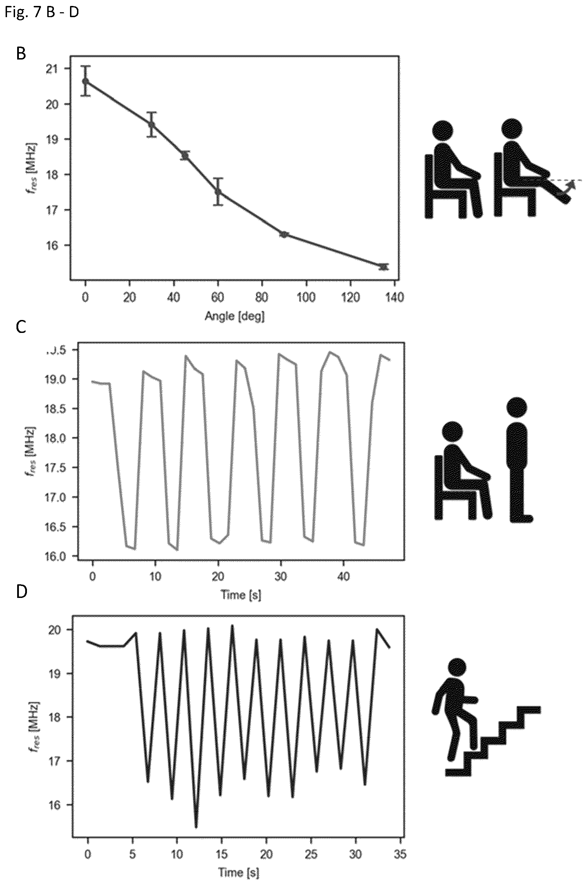

Figure 7 Demonstration of the sensing platform for motion tracking. A, Functionalized pants used for the activity monitoring and knee bending angle tests. B, Knee bending angle tracking (mean and standard deviation) - the angle was measured with a goniometer during the test and is indicated in the schematic in red. 5 tests were run for each angle. C, Sit to stand (squatting) monitoring - the resonance frequency as the frequency of the S_11 peak is reported. D, Stair climbing monitoring - the resonance frequency as the frequency of the S_11 peak is reported.

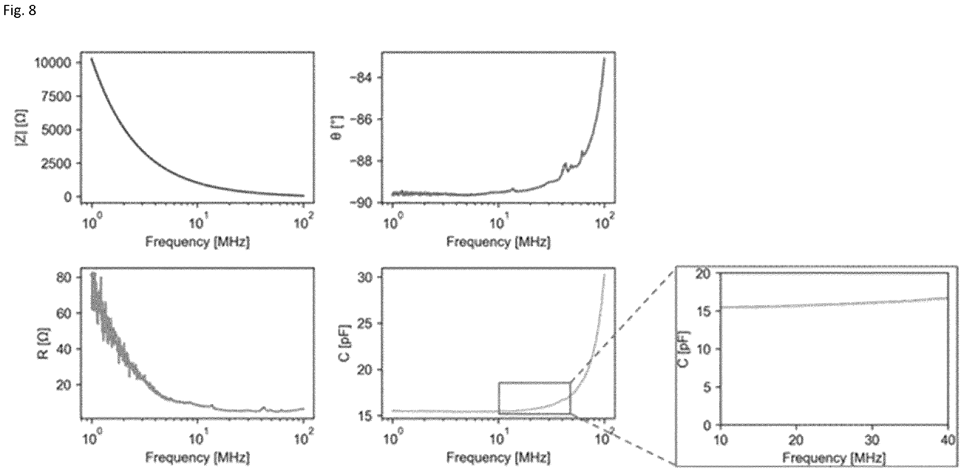

Figure 8 shows the impedance characterization of a textile capacitive sensor Cs. The textile capacitor displays capacitive behavior with "-88°<θ<-90°" until about f = 60 MHz. Above this frequency the capacitance starts changing and the phase shifts away from the ideal value of -90°. The self-resonance frequency is outside the frequency range 1-100 MHz. The zoom-in shows the capacitance in the frequency range of interest (around the theoretical resonance frequency) varying from 15.49 pF at 10MHz to 16.73 pF at 40 MHz.

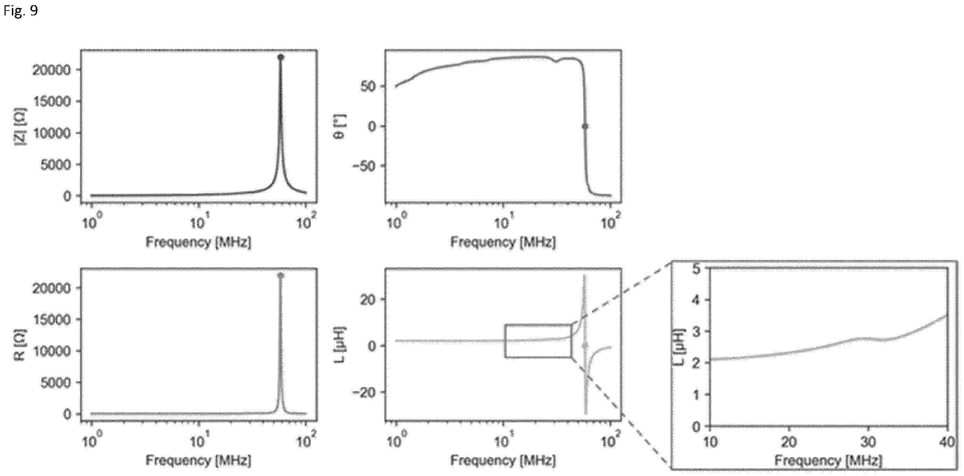

Figure 9 shows the impedance characterization of the textile inductor Ls. The textile presents its self-resonance frequency between 57.88 MHz and 58.21 MHz - a slight difference is present among different parameters. The zoom-in shows the inductance in the frequency range of interest (around the theoretical resonance frequency) varying from 2.07 $\mu$H at 10 MHz to 3.52 $\mu$H at 40 MHz.

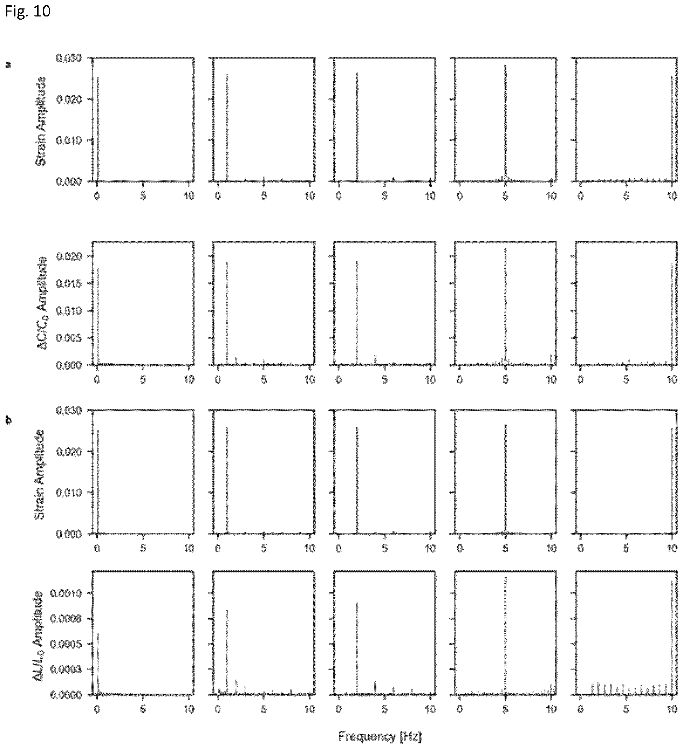

Fig. 10 shows the analysis of textile capacitor and inductor bandwidth in the frequency domain. Fast Fourier Transform (FFT) analysis of the electrical response to sinusoidal strain waves at increasing frequencies for the textile capacitive sensor (a) and the textile inductor (b). For f = 5 Hz and f = 10 MHz, subsets of the 30 sinusoidal waves displaying consistent amplitude were used for the analysis, in order to exclude the ramp-up and ramp-down time of the UTM and the waves with a non-consistent amplitude.

Fig. 11 a) calculated plots the different maximum strain accessible with respect to the helical length of the initial helical auxetic yarn capacitive sensor with two common ratio of diameters; b) calculated plots the Poisson ratio of the sensors with respect to the maximum strain accessible for each sensors shown in the (a) plot for two different ratio of diameters; c) experimentally obtained Gauge Factor obtained for specific helical lengths for sensors for two different diameters; d) the experimental data of strain to capacitance for 20:1 ratio sensors with various helical lengths (e.g. E3W1-8 = 20:1 with a helical length of 8mm).

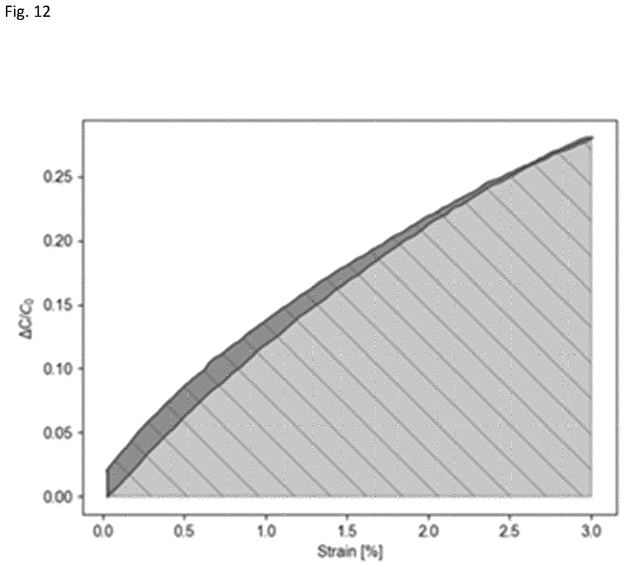

Fig. 12 shows the hysteresis test of the plate capacitor which was measured as the ratio between the area enclosed in the extension-relaxation loop divided by the area under the curve of the extension curve.

DESCRIPTION OF THE PREFERRED EMBODIMENTS

The inventors were able to produce an exemplary sensor using exclusively textile-based circuitry. The sensor was characterized, integrated in common clothing and as a proof-of-concept it was shown that it is capable of tracking human motion.

The design of the sensing platform was based on an LC circuit (Fig 1) composed of a textile capacitor sensitive to strain, a fixed value inductor, and an electrical connection between these two. The change in capacitance upon strain was tracked by measuring the resonance frequency of the circuit.

The inventors designed the textile capacitor and inductor to fit in the size of a pants. To manufacture the textile capacitor and inductor, the inventors sought materials with high conductivity, mechanical strength, natural textile feeling and washability. They chose spandex - also known by the commercial name Lycra® - as a base material, since it is commonly used in sportswear and more generally in form fitting garments. The stretchable capacitor was fabricated by employing a double plate capacitor configuration with electrodes made of conductive spandex separated by two layers of non-conductive spandex (Fig 1B). The inductor was fabricated by sewing a highly conductive thread in planar rectangular loops with equal spacing between each turn of the loop (Fig. 1B). The electrical connection between the inductor and the capacitor was produced by sewing conductive thread into the base spandex textile.

To predict the resonance frequency of the system at rest and under applied strain, the two components impedance response to strain was characterized. The baseline impedance of the unstretched sensor was measured across a frequency range of 1 MHz - 100 MHz. From the values of C and L, the theoretical resonance frequency can be calculated. The capacitor displayed a consistent baseline capacitance of 15.5 pF between 10 MHz and 40 MHz **(Figure 8)**; this value was close to the expected value. Whereas the inductor had a constant baseline inductance of 3 $\mu$H up in the lower frequency range up to 20 MHz **(Figure 9).** Thus, the impedance of the LC circuit with no strain was 23 MHz.

The response of both textile components to mechanical strain was evaluated by imposing displacement patterns with a universal testing machine and measuring the corresponding electrical change (C or L). Specifically, the sensing metrics characterized were strain-response (i.e. linearity), sensitivity, drift, and stability over prolonged repetitive strain. Analogous tests were performed on C and L to understand the response of each component to strain. The results are reported in and Fig. 4 and Fig. 5 respectively.

First, the mechanical response of the two textile components was measured with a standard tensile stress-strain test. This test serves to provide a basic relationship of the materials response (i.e. stress) to the applied strain; this would then allow us to deduce if the electrical response of our components was a result of the mechanical properties of the textiles or because of an electrical phenomenon. Both components displayed a linear stress response to strain.

From the same stress-strain test, the electrical response was also obtained. The maximum strain for the capacitor was 70%, calculated by the formula in Fig. 3a. Two regions of interest were observed: first, a steeper increase in capacitance up to 5-10% strain (Fig. 3b), and second above 10% strain. The gauge factor (*GF*)-or sensitivity-of the capacitive sensor was calculated resulting in a *GF* higher than 1 in the initial region and a *GF* of 1 ($R^2$ = 0.99) for above 10% strain. The $C_{WSD}$ displayed a higher sensitivity in the low strain range of interest (0-10%), which is particularly interesting to potentially detect small motions or bio-signals through textiles. Interestingly, in the low strain region (0-10%) the *GF* was above the theoretical limit of 1 for parallel plate capacitors where capacitance is proportional only to electrode area, which scales linearly with strain above 10% we observed a *GF* of 1.

Next, the consistency of the sensor was tested. Clothing may undergo strain dynamically (i.e. during movement) or statically (i.e. sitting) and therefore the sensor should have high consistency throughout different movements to achieve accurate body movement tracking. The static consistency of the sensor was evaluated with a step-hold test, observing any change in capacitance during each holding step at different strain values (Fig. 3e). The variation in the capacitance over a 50 second hold period was negligible (less than 0.4%) which is ideal for a wearable garment meant to accurately track body movement.

All materials possess some amount of mechanical hysteresis, while sensors may also possess an electrical hysteresis. Typically, hysteresis is evaluated by measuring the area enclosed in the stress strain loop (i.e. an ideal sensor would have zero area). As expected, the capacitive sensor presented an increasing hysteresis with increasing strain, but overall had low hysteresis (4.48% of the area under the curve of the extension curve at 30% strain). Low hysteresis ensures predictable behavior and repeatable correlation of signal-strain whether elongating or shortening and should therefore occur over repetitive cycles.

To analyze the long-term stability, the sensors were strained for 1000 cycles from 10-15% strain (i.e. within our intended working range). They displayed consistent capacitance-strain response over the 1000 cycles, with variation in the capacitance signal maxima (peaks) and the minima (valleys) within 1% of the starting values **(Fig. 3f,)**. These results are encouraging for applying these sensors into prolonged use since long-term signal drift and instability are major issues when developing wearable devices.

Lastly, wearable devices must reliably respond to various strain rates. Generally, a decrease in amplitude and a delay in the electrical response are expected as the loading frequency increases. The capacitive sensor showed ability to track strain without amplitude loss or signal lag up to the maximum tested frequency of 10 Hz, with a 0.02s lag in the capacitance response at 10 Hz **(Fig. 3d, Figure 10 a)**.

Analogous to the capacitor, the inductor was strained along the longer side of its rectangular shape **(Fig. 4a)** and displayed almost no variation in L (<4%) in the applied strain range, nor any correlation to the applied strain **(Fig. 4b)**, thus the GF could not be quantified, and we considered a *GF* of 0. A slightly higher static drift (up to 4%,) was observed as compared to the capacitor during the step-hold test **(Fig. 4e)**, low hysteresis **(Fig. 4c)**, and negligible dynamic drift **(Fig. 4f,)**. Lastly, the inductor showed a good response in at increasing strain rates with negligible time lag and small variation in the amplitude.

To track the resonance frequency of the LC system wirelessly, we employed a portable VNA. The resonance frequency was tracked by determining the minimum of the reflection coefficient profile defined as:

$$S_{11} = 10\,log_{10}\left(\frac{P_r}{P_i}\right) \qquad\qquad (1)$$

where $P_i$ denotes the incident power sent by the reader to the sensor and $P_r$ denotes the power reflected to the reader. When the reader sends a signal scanning all frequencies to the inductively coupled sensor coil, at the sensor resonance the minimum of $S_{11}$ denotes maximum electromagnetic energy absorption by the sensor. The interface between this device and the circuit was obtained by inductive coupling between the textile inductor and an external inductor-a rectangular coil of insulated single strand wire with 3 turns (Fig. 1A)-which was in turn connected to the VNA. The reader inductor had a slightly smaller size than the textile inductor such that the two could be placed concentrically and tolerate small misalignments in the in-plane direction. The two inductors were put in close proximity to each other in the axial direction to maximize their coupling. The influence of the relative position of the coupled inductors was evaluated in all three spatial directions **(Fig. 5a)**. As the distance between the two inductors increased, the $S_{11}$ profile became less sharp, and its absolute value decreased progressively. With the specific system tested, a vertical displacement of 50 mm resulted in complete signal loss. The system showed tolerance to vertical displacement up to 10 mm; even at 25 mm distance it was possible to track the $S_{11}$ signal, despite low intensity and sharpness as compared to a scenario with lower vertical distance. A misalignment in the-plane direction of up to half of the inductor size caused signal loss along the longer side of the rectangular planar inductor **(Fig. 5b)** but not on the short side **(Fig. 5c)**. Radial displacement of the two inductors seemed to have a higher impact on the frequency of the resonance peak as compared to the axial displacement.

Considering the observed influence of the relative displacement of the coupled inductors on the signal quality, the reader inductor was placed in a tight pocket on top of the textile inductor to mitigate any potential displacement.

To test the ability of the system to track motion in a wearable device, the all-textile LC circuit was integrated into tight fitting sport pants. A transmission line made of conductive thread served as connection between the two components to close the LC circuit. The capacitor was placed above the kneecap to track motion of the leg, whereas the inductor was placed within a pocket near the waist, such that the inductor could be placed in a pocket adjacent to the inductor **(Fig. 7A)**. The transmission line running vertically from the inductor down to the capacitor was sewn with a zigzag stitch to allow free movement of the leg and stretch of the transmission line if required. The influence of the transmission line length on the output signal ($S_{11}$) revealed a nonlinear relationship between increasing transmission line length and shift in resonance frequency. Nonetheless, for a fixed transmission line length the response was consistent.

First, the system was tested under static conditions by tracking the knee bending angle. The progressive stretching of the capacitor electrodes with increasing knee bending (30, 45, 60, 90 and 135 degrees) resulted in a clear decrease in the resonance frequency, showing the capability of the system to detect different degrees of bending **(Fig. 7B)**. The bending angle was manually measured with a goniometer taking the thigh inclination as reference (illustration in **Fig. 7B)** and the resonance frequency was determined as the minimum of the $S_{11}$ profile for each of 3 tests performed for each bending angle.

Next, we evaluated the dynamic performance of the system for motion tracking by performing common everyday activities such as sitting-to-standing (equivalent to a squat exercise) and stair climbing. The response of the system in terms of resonance frequency shift revealed the ability to track these motions, with a shift in resonance frequency of approximately 3 MHz when the knee was fully bent **(Fig. 7C, D)**. The system described here may be applied to any form fitting wearable device to monitor movement passively and wirelessly including areas of the body that would be impractical to use alternative available technologies (such as inertial measurement units, IMUs), such as the back when sitting. Further improvement of the presented technology is possible upon access to higher acquisition rate of the reader: this feature would allow to track faster activities (e.g. running) and generally increase the time resolution.

In conclusion, it was possible to develop a fully textile-based wireless sensor for tracking body movement. The sensor is based on a passive LC circuit resonance and tracks movement by monitoring the resonance frequency of the circuit, which is modulated through the change in capacitance.

Methods and Material

Electrolycra® 234-309A was purchased from MindSets (Saffron Walden, UK). Yellow spandex textile with one way stretch was purchased from Spitzenraum (Schänis, Switzerland). Liberator® thread was purchased from Syscom Advanced Materials (Columbus, OH, United States). Sewing was completed on a Singer Heavy Duty machine (Model 4423). Conductivity measurements were completed with a digital multimeter (Fluke 177, Everett, Washington, USA). Impedance measurements of the unstretched samples were performed with a HIOKI IM7581-1 impedance analyser (Ueda, Nagano, Japan). Tensile tests were completed on an Instron E3000 universal tensile measurement (UTM) device (Norwood, MA, United States). Impedance measurements during the tensile tests were performed with a precision inductance-capacitance-resistance (LCR) meter - Hioki IM3536 LCR (Ueda, Nagano, Japan) with four lead probe setups. For tensile - electronic measurements the LCR meter was interfaced with a PC using a custom LabView script and synchronized with the UTM with a Data Acquisition module (National Instruments, Austin, TX, US). Vector Network Analyzer measurements were completed using a miniVNA pro (MiniRadioSolutions, Herxheim, Germany) and a custom inductor coil from insulated copper wire (22G) with 3 rectangular loops.

The capacitor of the sensor was arranged in a double plate configuration by stitching two conductive stretchable textiles 50 mm x 35 mm on either side of double-layered yellow spandex textile (Fig. 1). The conductive stretchable textiles were sewn to the base yellow spandex in a taut configuration-to avoid wrinkling-and such that they would precisely overlap in area. Care was taken to avoid shorting between the two sheets during sewing. The DC resistance was measured with a digital multimeter on multiple square samples resulting in a mean value of 1.0 Ω/cm (from the manufacturer: 0.5 Ω/cm, increasing to 1.33 Ω/cm when stretched in one axis but dropping to 0.16 Ω/cm when stretched at 90° to this).

The textile inductor was manufactured by sewing conductive Liberator® thread on a piece of yellow spandex fabric with a standard sewing machine. The thread was sewn in a rectangular loop with dimensions 80 mm x 60 mm, with 5 turns and 2 mm gap between the turns (Fig. 1B, $L_{WSD}$). A consistent gap between the loops was ensured during fabrication. A wash-away sewing stabilizer was used to avoid wrinkles and produce a flat textile inductor. The highly conductive and mechanically stiff thread was used to minimize resistance and ensure mechanical robustness. The DC resistance of the thread was measured with a digital multimeter for 1 cm long samples resulting in a mean value of 0.036 Ω/cm (3.3 Ω/m according to the manufacturer).

The baseline impedance of the stretchable capacitor and textile inductor was measured across a frequency range from "1 MHz" to 100 MHz with an impedance analyzer. The overall impedance, phase, capacitance (or inductance) and resistance were measured to characterize the electrical behavior of each component of the circuit. The resistance of the overall circuit was measured with a digital multimeter.

The electrical behavior of the sensors during the tests was acquired with a precision LCR meter with four lead probe setups. The electrically conductive thread terminals of the samples were clipped to the LCR meter probes. The meter was configured to track impedance magnitude and phase, ESR, capacitance and inductance.

A tensile stress strain test was conducted between 0% and maximum attainable strain corresponding to the maximum stroke of the machine head (70% for the capacitor, 80% for the inductor) at a strain rate of 1%/s. The Young's modulus of the samples was calculated as the slope of the linear fit to the data. Similarly, the linearity of the electrical response and the GF were obtained from this test.

Hysteresis test consisted of three triangular waves at a strain rate of 1%/s with maximum strain of 10%, 20% and 30%. This resulted in a closed loop curve made up by forward (extension) and backward (relaxation) curves. An ideal sensor with no hysteresis gives the same response when extended and relaxed. Hysteresis is defined as the area enclosed in the closed curves (extension-relaxation), with the ideal case being area=0. The hysteresis was measured as the ratio between the area enclosed in the extension-relaxation loop divided by the area under the curve of the extension curve (Figure 12).

For a bandwidth test, 30 sinusoidal waves of increasing frequency at the same strain amplitude (2.5%) and a prestrain of 7.5% were applied. The ability of the textile component to track the strain sinusoidal wave without amplitude loss or strain-electrical signal lag was used as evaluation metric. This was assessed by an analysis in the frequency domain.

A step-hold strain pattern consisting of three steps from 0 to 30% (10% step) at a strain rate of 1%/s was applied; the ramp up and ramp down phases were 10 seconds long, the hold phase 50 seconds. The variation in the electrical response during the hold phase was evaluated as variation with respect to the baseline (first value of the hold phase). The long-term stability was tested for the evaluation of dynamic drift by imposing 1000 sinusoidal waves at "5%" strain (f=1 Hz) with 10% prestrain. The variation of peaks and valleys of the electrical response compared to the values in the first strain cycle was used as a metric.

A reader inductor was fabricated with single strand insulated wire arranged in a planar rectangular loop (3 turns) with dimensions were 80 mm x 60 mm to match the sensor textile inductor. The impedance was measured in the same frequency range utilised to characterise the textile components, i.e. from 1 MHz to 100 MHz (Supplementary Figure 3). The parasitic capacitance was calculated based on the measured self-resonance frequency and the commonly used electrical equivalent circuit for such inductors (Supplementary Figure 4).

The readout relied on inductive coupling between the sensor inductor and reader inductor. The optimal configuration is a concentric and parallel position; increasing the vertical distance between or the displacing one laterally along the same plane decreases the coupling strength. The influence of relative displacement of the two planar inductor was investigated by acquiring the signal in different configurations, i.e. displacing the reader inductor with respect to the sensor inductor in all three directions as displayed in Fig. 5. Of note, the multiturn configuration for the textile inductor $L_{WSD}$ (5 turns, 2 mm gap between the turns) also resulted in better tolerance to horizontal misalignment between the textile inductor and reader inductor.

The capacitor and textile inductor were connected to form the LC circuit with the same conductive thread used to manufacture the inductor. First, this thread was attached to each of the two electrodes of the capacitor. Of note, the textile inductor was placed in an anatomical location where limited strain would take place.

Knee bending angle and motion tracking were tested: The reader inductor was connected to a portable vector network analyzer (miniVNA) that detected the resonance frequency of the LC resonator in real time. The miniVNA was set to scan a frequency range between 15 MHz and 25 MHz as this range was around the theoretical resonance frequency of the LC circuit and allowed for faster continuous sweeping as there is a trade-off between resolution in time and frequency range. The miniVNA reader could also be operated by the smartphone through the BlueVNA app available on Playstore. For the knee bending tests, three tests were performed for each angle measured with a goniometer (Fig. 7 B).

The reagents used for the fabrication and evaluation of helical auxetic yarn were purchased from Sigma Aldrich and used as received unless otherwise stated. The base elastic fiber (800 $\mu$m diameter; PET wound elastomer/rubber) was purchased from Spitzentraum (Zurich, Switzerland) and used as received. The copper wires with enamel coatings used as electrodes were purchased from Digitec-Galaxus (xx $\mu$m diameter; Switzerland). Inductance-capacitance-resistance (LCR) measurements were made with an Hioki IM 3536 (Linktronix; Switzerland). The tensile testing was completed on an Instron E3000.

Auxetic helical yarns were fabricated as follows: The inner elastic polypyrrole fiber electrodes (PPy EF) were fabricated by first, soaking a length of elastic fiber (EF) in a solution of $FeCl_3$ in methanol (3M) for 15 minutes then air dried. The $FeCl_3$ impregnated EF was then inserted into a glass desiccator with an open volume of pyrrole (1 mL). The vapor polymerization was allowed to proceed for 15 minutes, at which time it was removed and repetitively stretched to the fibers maximum strain (ca. 100%). The EF was then inserted back into the closed desiccator with the open volume of pyrrole and allowed to continue vapor phase polymerization; this removal-stretch-reinsertion was completed a total of four times. The vapor phase polymerization was then allowed to proceed overnight. The EF was then removed and the residual $FeCl_3$ was then removed from the PPy-EF by soaking the fiber in methanol and water. The conductive PPy-EF fibers were then air dried and kept in a sealed bag until use.

The auxetic capacitive sensors were fabricated by tying an overhand knot at each end of the PPy-EF (1 or 3 PPy-EF). The copper wire was then manually wound around the PPy-EF ensuring that the wire had a consistent pitch and then secured on the outer end of each PPy-EF knot with three half hitches. On one end of the excess copper wire, the enamel was removed using 150 grit sandpaper where the alligator clip of the LCR was attached, with the other alligator clip attached to the PPy-EF.

The helical auxetic capacitive sensors were tested on a tensile tester (Instron E3000) and custom design 3D printed grippers (see SI for images). An LCR was used to monitor the sensors output by attaching one lead to the exposed copper wire and the other a free end of the PPy-|EF electrode. Series capacitance, series resistance, phase angle, and impedance were measured during all tensile testing at a sampling frequency of 1 kHz and at a strain rate of 1mm/s.

The resulting signal files from the tensile tester and LCR device were resampled to 100 Hz. This resample was completed to allow the comparison of strain to capacitance directly. The files were synced using a custom software developed within our group specifically for the Hioki LCR and Instron E3000 equipment.

The auxetic character of the helical auxetic yarn sensors were initially predicted using only two states-the initial and final states. It was assumed that the number of helices in the initial and the final state would be equal and that the PPy-EF had a Poisson's ratio of 0.4. Employing trigonometry of a helix around a circle, the inventors were able to determine the length of the rigid fiber, and therefore the maximum strain. This was completed for a range of pitches (see SI for table), for which one can calculated the final length of the PPy-EF and its final diameter using the equation for larger strains. The cross-sectional area of the helical auxetic yarn sensors were then calculated by adding up one inner electrode and two outer electrode diameters in the initial state and the final state. This enables a crude estimation of where auxetic character would occur if the helical auxetic yarn sensors were able to access the maximum strain (i.e. RF).

## Claims

1. A sensor for detection of strain comprising a LC circuit and being made using exclusively textile components.

2. The sensor according to claim 1, wherein the sensor is flexible.

3. The sensor according to claim 1 or 2, wherein the inductor of the LC circuit comprises a sewn pattern of a conductive thread or is made entirely of the sewn pattern of a conductive thread.

4. The sensor according to anyone of claims 1 - 3, wherein the capacitor of the LC circuit has a double plate capacitor configuration.

5. The sensor according to claim 4, wherein the capacitor consists of two conductive, flexible sheets of a textile material separated by at least one layer of a non-conductive flexible textile material.

6. The sensor according to any one of claims 1 - 5 being arranged to allow wireless readout.

7. The sensor according to anyone of claim 1 - 3 or 6, wherein the capacitor of the LC circuit comprises a helical auxetic yarn.

8. The sensor according to claim 7, wherein the helical auxetic yarn is made from at least two conductive fibers twisted around one another and insulated from one another and wherein the first fiber of the at least two conductive fibers is non-extending and the second fiber of the at least two conductive fibers is elastic.

9. The sensor according to claim 7 or 8 having a gauge factor greater than 1.

10. The sensor according to claim 8 or 9, wherein the ratio of diameter between the first and the second fiber is between 1:5 and 1:50.

11. The sensor according to anyone of claims 8 - 10, wherein the twisting angle of the first and the second fiber is < 40°.

12. Use of the sensor according to any one of claims 1 - 11 for tracking human motion.

13. A system comprising the sensor according to any one of claims 1 - 12 and a transponder unit configured to measure a frequency response of the sensor.

14. A garment including the sensor according to any one of claims 1 to 11.

15. The garment according to claim 14 further comprising a transponder unit configured to measure a frequency response

of the sensor.

Fig. 1

Fig. 2

A

B

Fig. 3

Fig. 4

Fig. 5

Fig. 6

Initial (Helical) State
$\varepsilon_i$

HL
$D_R$
$D_E$
$\theta_i$
$L_E$

Twisted State
$\varepsilon_t$

$\theta = \phi$

Final (Auxetic) State
$\varepsilon_f$

$\phi_f$
$L_R$

Fig. 7

A

Fig. 7 B - D

Fig. 8

Fig. 9

Fig. 10

Fig. 11

**a.**

Pitch—Max Strain

**b.**

Auxetic Character—Max Strain

Fig. 11

**c.**

Pitch — Gauge Factor

**d.**

Fig. 12

Europäisches
Patentamt

European
Patent Office

Office européen
des brevets

# EUROPEAN SEARCH REPORT

**Application Number**

EP 22 20 7284

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/303383 A1 (CONNOR ROBERT A [US]) 25 October 2018 (2018-10-25) | 1-6, 12-15 | INV. G01B7/16 |
| Y | * abstract * * paragraphs [0431], [0474], [0220]; claim 1 * | 7-11 | A61B5/11 A61B5/00 D03D1/00 G01L1/14 |
| X | US 2022/054041 A1 (MENON CARLO [CA] ET AL) 24 February 2022 (2022-02-24) | 1-6, 12-15 | G01L1/22 G01L9/12 |
| Y | * claim 1; figures 1,7 * | 7-11 | G01P15/125 G01R15/18 |
| Y | WO 2014/204323 A1 (STRETCHSENSE LTD [NZ]) 24 December 2014 (2014-12-24) * abstract; figure 18 * | 1-15 | |
| Y | CN 111 509 375 A (SHUMA CLOTHING CO LTD) 7 August 2020 (2020-08-07) * claim 1; figures 4-6 * | 1-15 | |
| X | US 2011/128686 A1 (MORESHEAD WYLIE [US]) 2 June 2011 (2011-06-02) | 1-6, 12-15 | |
| Y | * paragraph [0040]; figures 1,2,9 * | 7-11 | **TECHNICAL FIELDS SEARCHED (IPC)** |
| X | US 2008/218180 A1 (WAFFENSCHMIDT EBERHARD [DE] ET AL) 11 September 2008 (2008-09-11) | 1-6, 12-15 | G01B A61B |
| Y | * claim 1; figures 2a,3 * | 7-11 | G01R G01L |
| X | US 2011/127248 A1 (MORESHEAD WYLIE [US]) 2 June 2011 (2011-06-02) | 1-6, 12-15 | G01P D03D |
| Y | * paragraph [0040]; figures 1,2,9 * | 7-11 | |
| X | GB 1 596 298 A (MORGAN LTD P K) 26 August 1981 (1981-08-26) | 1-6, 12-15 | |
| Y | * claim 19; figures 1A-1C * | 7-11 | |

-/--

The present search report has been drawn up for all claims

| Place of search | Date of completion of the search | Examiner |
|---|---|---|
| Munich | 12 May 2023 | Pavón Mayo, Manuel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

# EUROPEAN SEARCH REPORT

**Application Number**

EP 22 20 7284

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LINA M CASTANO ET AL: "Smart fabric sensors and e-textile technologies: a review", SMART MATERIALS AND STRUCTURES, IOP PUBLISHING LTD., BRISTOL, GB, vol. 23, no. 5, 1 April 2014 (2014-04-01), page 53001, XP020262301, ISSN: 0964-1726, DOI: 10.1088/0964-1726/23/5/053001 [retrieved on 2014-04-01] | 1-6, 12-15 | |
| Y | * point 4.2 and point 4.1.1; page 22 * | 7-11 | |
| Y | WO 2021/113864 A1 (UNIV MINNESOTA [US]; US GOV ADMINISTRATOR OF NASA [US]) 10 June 2021 (2021-06-10) * page 11, line 12ff * | 7-11 | |
| X | Tae-Ho Kang: "Textile -embedded sensors for wearable physiological monitoring systems", , 19 May 2007 (2007-05-19), pages 1-144, XP055402313, ISBN: 978-0-549-20213-4 Retrieved from the Internet: URL:https://repository.lib.ncsu.edu/bitstream/handle/1840.16/5143/etd.pdf?sequence=1&isAllowed=y [retrieved on 2017-08-30] | 1-6, 12-15 | |
| Y | * top half of page 126 and FIG. 8 * | 7-11 | **TECHNICAL FIELDS SEARCHED (IPC)** |

-/--

The present search report has been drawn up for all claims

| Place of search | Date of completion of the search | Examiner |
|---|---|---|
| Munich | 12 May 2023 | Pavón Mayo, Manuel |

EPO FORM 1503 03.82 (P04C01)

Europäisches
Patentamt

European
Patent Office

Office européen
des brevets

# EUROPEAN SEARCH REPORT

Application Number

EP 22 20 7284

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SHAYAN SEYEDIN ET AL: "Textile strain sensors: a review of the fabrication technologies, performance evaluation and applications", MATER. HORIZ., vol. 6, no. 2, 1 January 2019 (2019-01-01), pages 219-249, XP055719454, ISSN: 2051-6347, DOI: 10.1039/C8MH01062E | 1-6, 12-15 | |
| Y | * title, page 246 at the paragraph bridging left and right column and page 242 left column at the bottom * ----- | 7-11 | |
| X | WO 2022/182297 A1 (UNIV SINGAPORE TECHNOLOGY & DESIGN [SG]) 1 September 2022 (2022-09-01) | 1-6, 12-15 | |
| Y | * par. 77 and FIG. 32 * ----- | 7-11 | |
| X | MATTEO STOPPA ET AL: "Wearable Electronics and Smart Textiles: A Critical Review", SENSORS, vol. 14, no. 7, 7 July 2014 (2014-07-07), pages 11957-11992, XP055327328, DOI: 10.3390/s140711957 | 1-6, 12-15 | |
| Y | * strech sensors at 2.5.1 and pressure sensors at 2.5.2, FIG. 12 and 13 and reference to textile RFID at page 11974 * ----- | 7-11 | TECHNICAL FIELDS SEARCHED (IPC) |
| X | US 2016/000374 A1 (DANDEKAR KAPIL R [US] ET AL) 7 January 2016 (2016-01-07) | 1-6, 12-15 | |
| Y | * claim 1 + 9 and FIG. 5 and par.5 * ----- | 7-11 | |
| X | US 2022/183627 A1 (SASSI UGO [GB] ET AL) 16 June 2022 (2022-06-16) | 1-6, 12-15 | |
| Y | * FIG. 8B and par. 261-262 * ----- | 7-11 | |

-/--

The present search report has been drawn up for all claims

| Place of search | Date of completion of the search | Examiner |
|---|---|---|
| Munich | 12 May 2023 | Pavón Mayo, Manuel |

EPO FORM 1503 03.82 (P04C01)

Europäisches
Patentamt

European
Patent Office

Office européen
des brevets

# EUROPEAN SEARCH REPORT

Application Number

EP 22 20 7284

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RONGZHOU LIN ET AL: "Wireless battery-free body sensor networks using near-field-enabled clothing", NATURE COMMUNICATIONS, vol. 11, no. 1, 23 January 2020 (2020-01-23), XP055766311, DOI: 10.1038/s41467-020-14311-2 * title and FIG. 1-2 for example * | 1-15 | |
| Y | SHUKLA SHIVANGI ET AL: "Auxetic fibrous structures and their composites: A review", COMPOSITE STRUCTURES, ELSEVIER SCIENCE LTD, GB, vol. 290, 1 April 2022 (2022-04-01), XP087031966, ISSN: 0263-8223, DOI: 10.1016/J.COMPSTRUCT.2022.115530 [retrieved on 2022-04-01] * abstract and reference to helical auxetic yarns at point 5.3 * | 7-11 | |
| Y | GAO YAJIE ET AL: "A Study of Woven Fabrics Made of Helical Auxetic Yarns", APPLIED COMPOSITE MATERIALS, KLUWER ACADEMIC PUBLISHERS, LONDON, NL, vol. 29, no. 1, 6 October 2021 (2021-10-06), pages 109-119, XP037746080, ISSN: 0929-189X, DOI: 10.1007/S10443-021-09952-5 [retrieved on 2021-10-06] * abstract * | 7-11 | TECHNICAL FIELDS SEARCHED (IPC) |

-/--

The present search report has been drawn up for all claims

| Place of search | Date of completion of the search | Examiner |
|---|---|---|
| Munich | 12 May 2023 | Pavón Mayo, Manuel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................

& : member of the same patent family, corresponding
document

EPO FORM 1503 03.82 (P04C01)

Europäisches
Patentamt

European
Patent Office

Office européen
des brevets

## EUROPEAN SEARCH REPORT

**Application Number**

EP 22 20 7284

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHANG YUPING ET AL: "3D Fabrics with Negative Poisson's Ratio: A Review", APPLIED COMPOSITE MATERIALS, KLUWER ACADEMIC PUBLISHERS, LONDON, NL, vol. 29, no. 1, 9 November 2021 (2021-11-09), pages 95-108, XP037746086, ISSN: 0929-189X, DOI: 10.1007/S10443-021-09931-W [retrieved on 2021-11-09] * abstract and reference to helical auxetic yarns at, eg, the conclusion at page 106 * | 7-11 | |
| | | | **TECHNICAL FIELDS SEARCHED (IPC)** |

The present search report has been drawn up for all claims

| Place of search | Date of completion of the search | Examiner |
|---|---|---|
| Munich | 12 May 2023 | Pavón Mayo, Manuel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 20 7284

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2023

| Patent document cited in search report | | Publication date | Patent family member(s) | | Publication date |
|---|---|---|---|---|---|
| US 2018303383 | A1 | 25-10-2018 | NONE | | |
| US 2022054041 | A1 | 24-02-2022 | NONE | | |
| WO 2014204323 | A1 | 24-12-2014 | NONE | | |
| CN 111509375 | A | 07-08-2020 | NONE | | |
| US 2011128686 | A1 | 02-06-2011 | NONE | | |
| US 2008218180 | A1 | 11-09-2008 | CN | 101218513 A | 09-07-2008 |
| | | | EP | 1904860 A1 | 02-04-2008 |
| | | | JP | 2009501040 A | 15-01-2009 |
| | | | US | 2008218180 A1 | 11-09-2008 |
| | | | WO | 2007007217 A1 | 18-01-2007 |
| US 2011127248 | A1 | 02-06-2011 | NONE | | |
| GB 1596298 | A | 26-08-1981 | GB | 1596298 A | 26-08-1981 |
| | | | JP | S5922523 B2 | 28-05-1984 |
| | | | JP | S53126786 A | 06-11-1978 |
| | | | US | 4308872 A | 05-01-1982 |
| | | | US | 4815473 A | 28-03-1989 |
| WO 2021113864 | A1 | 10-06-2021 | US | 2023002937 A1 | 05-01-2023 |
| | | | WO | 2021113864 A1 | 10-06-2021 |
| WO 2022182297 | A1 | 01-09-2022 | NONE | | |
| US 2016000374 | A1 | 07-01-2016 | US | 2016000374 A1 | 07-01-2016 |
| | | | WO | 2014138204 A1 | 12-09-2014 |
| US 2022183627 | A1 | 16-06-2022 | EP | 3736528 A1 | 11-11-2020 |
| | | | US | 2022183627 A1 | 16-06-2022 |
| | | | WO | 2020225039 A1 | 12-11-2020 |

EPO FORM P0459

For more details about this annex : see Official Journal of the European Patent Office, No. 12/82